(19) Europäisches Patentamt
European Patent Office
Office européen des brevets

(11) **EP 4 338 770 A1**

(12) **EUROPEAN PATENT APPLICATION**

(43) Date of publication:
**20.03.2024 Bulletin 2024/12**

(21) Application number: **22195657.6**

(22) Date of filing: **14.09.2022**

(51) International Patent Classification (IPC):
**A61M 5/142** (2006.01)    **A61M 5/145** (2006.01)
**A61M 5/168** (2006.01)

(52) Cooperative Patent Classification (CPC):
**A61M 5/14526; A61M 5/14248;** A61M 2005/14268;
A61M 2005/16863; A61M 2205/3561;
A61M 2205/52

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Sensile Medical AG
4600 Olten (CH)**

(72) Inventors:
• **LAGORGETTE, Pascal
2502 Bienne (CH)**
• **BÜRLI, Fabian
4655 Stüsslingen (CH)**
• **MÜLLER, Matthias
4614 Hägendorf (CH)**
• **KOSTAL, Peer
D-79618 Rheinfelden (DE)**

(74) Representative: **reuteler & cie SA
Chemin de la Vuarpillière 29
1260 Nyon (CH)**

(54) **DRUG DELIVERY DEVICE**

(57)    A drug delivery device (1), comprising a delivery unit (3) including a drug container (6) comprising a barrel portion (6a) and a plunger (12) slidably mounted within the barrel portion and sealing the drug (78) within the container at one end of the barrel portion, and a drive unit (4) comprising a pneumatic flow system (74) and a pneumatic pumping system configured to pump air via the pneumatic flow system (74) to a space behind the plunger to generate a gas pressure to advance the plunger in the container during drug delivery. The drug delivery device comprises a sealing adaptor (9) mounted on an end of the drug container facing an outer side of the plunger, the sealing adaptor (9) comprising a sealing plug (23) inserted inside an end of the barrel portion, the sealing plug (23) comprising an orifice (26) configured for pluggable sealing connection to a gas channel connector (40) of the pneumatic flow system.

FIG 2a

EP 4 338 770 A1

**Description**

## TECHNICAL FIELD

This invention relates to a drug delivery device for subcutaneous administration of a liquid drug. The invention in particular relates to a drug delivery device in the form of a patch device.

## DESCRIPTION OF RELATED ART

Drug delivery devices in the form of a patch device for mounting on a patient's skin for subcutaneous delivery of liquid drug are known. It is known to provide drug delivery devices in the form of a patch device with a single use disposable component assembled to a reusable component containing drive and control electronics, or as a single disposable component. Some devices typically receive a cartridge or have an internal reservoir that is filled by the patient / healthcare professional. In this case, the drug is drawn from a vial and transferred into the internal reservoir using a syringe. Since cartridges are widespread and their handling is so much easier, it is advantageous to provide a device that can be employed with standard cartridges.

The liquid drug may for instance be a biological medical product or other drugs that are administered merely for single shot administration, within a rather short time depending on the intended use.

In order to satisfy safety and reliability requirements, many conventional patch pump drug delivery devices have complex pump mechanisms and are rather bulky. The reliability, safety, compactness and ease of use of drug delivery devices worn by a patient is important, however for single shot administration, there is a reduced need for complex motor systems since one does not need to ensure accurate intermittent bolus or basal administration over a prolonged period extending over days or weeks. For disposable components, the amount of parts and consequently cost of the disposable device is also an important consideration. Conventional patch pumps to be worn by a patient are typically too complex and costly for single shot administration applications.

## SUMMARY OF THE INVENTION

In view of the foregoing, it is an object of the invention to provide a drug delivery device, in particular in the form of a patch device, with a disposable unit, or as an entirely disposable device, for single shot administration of a liquid drug that is safe, reliable, and compact.

It is advantageous to provide a drug delivery device that may be used for administration of liquid drugs provided in a drug container with a plunger.

It is advantageous to provide a drug delivery device that is easy to use.

It is advantageous to provide a drug delivery device that is economical to produce.

It is advantageous to provide a drug delivery device that has a long shelf life

Objects of the invention have been achieved by providing the drug delivery device according to claim 1. Dependent claims set forth various advantageous embodiments of the invention.

Disclosed herein is a drug delivery device, comprising a delivery unit including a drug container comprising a barrel portion and a plunger slidably mounted within the barrel portion and sealing the drug within the container at one end of the barrel portion, and a drive unit comprising a pneumatic flow system and a pneumatic pumping system configured to pump air via the pneumatic flow system to a space behind the plunger to generate a gas pressure to advance the plunger in the container during drug delivery.

The drug delivery device comprises a sealing adaptor mounted on an end of the drug container facing an outer side of the plunger, the sealing adaptor comprising a sealing plug inserted inside an end of the barrel portion, the sealing plug comprising an orifice configured for pluggable sealing connection to a gas channel connector of the pneumatic flow system.

In an advantageous embodiment, the gas channel connector comprises a tubular end portion pluggably inserted in the orifice of the sealing plug.

In an advantageous embodiment, the sealing plug comprises an inner radial sealing ring comprising a portion extending axially from an end wall of the sealing plug, said orifice for sealing connection to the gas channel connector being formed within the inner radial sealing ring.

In an advantageous embodiment, the plunger comprises a back end forming a cavity, said axial extension of the inner radial sealing ring being positioned at least partially in the cavity in an initial position prior to drug delivery.

In an advantageous embodiment, the sealing plug comprises an outer radial sealing ring comprising a portion extending axially from an end wall of the sealing plug, the outer radial sealing ring sealingly engaging an inner surface of the barrel portion of the drug container.

In an advantageous embodiment, the sealing adaptor comprises a cap inserted over an end of the barrel portion, the cap comprising an end wall engaging a back end of the sealing plug and one or more locking members engaging a housing component of the delivery unit in which the drug container is received.

In an advantageous embodiment, the housing component of the delivery unit is a container casing within which the drug container is inserted in the delivery unit, the locking member of the sealing adaptor cap engaging the complementary locking member on the container casing.

In an advantageous embodiment, the one or more locking members on the cap comprises one or more elastic latches having a locking shoulder engaging a complementary locking shoulder on the outside of the container casing.

In an advantageous embodiment, the cap of the sealing adaptor comprises a circular channel between an outer ring wall and an inner ring wall, the outer ring wall engaging over an outer end of the barrel portion of the drug container and the inner radial wall forming a space with the wall of the drug container within which an outer radial sealing ring of the sealing plug is lodged.

In an advantageous embodiment, the pneumatic flow system comprises a channel housing component within which a gas channel is formed extending between the gas channel connector and the outlet of the pump engine.

In an advantageous embodiment, the gas channel housing component is formed as a separated part assembled in the drive unit to the pump engine.

In an advantageous embodiment, the pneumatic pumping system comprises a pump engine including

a stator,

a rotor rotatably and axially slidably mounted at least partially in the stator, the rotor comprising a first axial extension having a first diameter and the second axial extension having a second diameter greater than the first diameter,

a first valve formed by a first valve seal mounted on the stator around the first axial extension, in conjunction with a first channel in the rotor that is configured to allow fluidic communication across the first valve seal when the first valve is in an open position, and

a second valve formed by a second valve seal mounted on the stator around the second axial extension, in conjunction with a second channel in a rotor that is configured to allow fluidic communication across the second valve seal when the second valve is an open position.

In an advantageous embodiment, the drug container is a drug cartridge and comprises a septum at one end.

In an advantageous embodiment, the drive unit comprises an electronic control system and a pressure sensor fluidically coupled to pneumatic flow system for measuring a gas pressure behind the plunger, the pressure sensor connected to the electronic control system of the drive unit configured to measure a pressure detected by the pressure sensor over time and to determine from the pressure measurement over time a position of the plunger over time, including a stop in movement of the plunger either due to occlusion in the drug delivery flow system or an end of travel of the plunger within the container corresponding to a container empty position.

Further objects and advantageous features of the invention will be apparent from the claims, from the detailed description, and annexed drawings, in which:

**BRIEF DESCRIPTION OF THE DRAWINGS**

Figure 1 is a perspective view of a drug delivery device according to an embodiment of the invention, showing a disposable delivery unit, a drug container, and a reusable unit in a disassembled state;

Figure 1a is a perspective view of the drug container with sealing adaptor mounted thereon of the device of figure 1;

Figure 2a is a perspective view of a portion of the drug delivery device according to an embodiment of the invention, showing a drug container housing part, sealing adaptor, and pneumatic flow system;

Figure 2b is an exploded view of figure 2a;

Figure 2c is a cross-sectional view through a plane 2c-2c of figure 2a;

Figures 3a to 3d are cross-sectional views of a portion of the drug delivery device showing a drug container housing part, drug container, sealing adaptor, and pneumatic flow system according to an embodiment of the invention, figure 3a showing the device prior to coupling of the disposable and reusable parts, figure 3b showing the device coupled; figure 3c showing the device in use during drug delivery, and figure 3d showing the device after drug delivery with the reusable and disposable units separated;

Figure 4 is a view similar to figure 3a of another embodiment;

Figure 5a is a schematic illustration of a drug delivery device according to an embodiment of the invention illustrating a pneumatic drive and a pneumatic plunger sensing system;

Figure 5b is a schematic illustration of a plot of pressure as a function of time of the pneumatic plunger system according to an embodiment of the invention;

Figure 5c is a schematic illustration of a plot of flow rates of a drug over time measured by a pneumatic plunger sensing system according to an embodiment of the invention;
Figure 5d is a corresponding plot of an air pumping pressure over time in the pneumatic flow system of the drug delivery device;
Figure 5e is a detail portion of the plot of air pumping pressure.

## DETAILED DESCRIPTION OF EXEMPLARY EMBODIMENTS

Referring to the figures, a drug delivery device 1 according to embodiments of the invention comprise a housing 2, a delivery unit 3, and a control or drive unit 4, the delivery unit 3 and the control or drive unit 4 being assembled within the housing 2. The housing 2 may be made of two or more parts allowing assembly of the delivery unit, drive unit and any other components within the housing.

In the illustrated embodiments, the drug delivery device for subcutaneous administration of a liquid drug (medicament) comprises a disposable portion formed by the delivery unit that may be assembled to a reusable portion formed by the drive unit, which includes electronics and a power supply. The delivery unit 3 is mounted in a first housing portion of the drug delivery device and the drive unit 4 in a separable second housing portion such that the drive unit 4 can be reused with subsequent delivery units.

Although the embodiments shown in the figures concern a two-part drug delivery device with disposable and reusable units, within the scope of the invention for various aspects described herein, the drug delivery device may be a single use disposable unit. The administration may occur in a single dose over a short period of time, typically less than 1 hour, for instance around 30 minutes or less. A single use disposable drug delivery device may also be used for subcutaneous injection of a liquid drug over an extended period of time from a few hours to a few days. Depending on the volume of the drug to be injected, the drug delivery device may also be configured to inject the liquid drug within a few minutes.

The drug delivery device includes a user interface 55 that may include one or more buttons for actuating the drug delivery device, light and/or sound status indicators, and optionally a screen or other display for presenting information to an operator of the device.

Drug delivery devices according to embodiments of the invention may advantageously be configured as a patch device for mounting on a patient's skin. An adhesive layer may be provided on an outer surface of a skin contact wall 81 of the housing 2 covered by a protective film 11 that may be peeled off the adhesive layer prior to placing the adhesive layer on the patient's skin at the site of injection. A needle orifice on the skin contact side is covered by the protective film 11 prior to use, and allows a transcutaneous injection needle (not visible) to extend therethrough and pierce the patent's skin upon activation of the drug delivery device 1.

The delivery unit 3 comprises a drug container 6, for instance a drug cartridge, containing a liquid drug 78, a liquid flow system for channeling the liquid drug to a patient subcutaneously, and a sealing adaptor 9 configured to enclose a back end of the drug container in a hermetic manner such that a gas pressure within the drug container portion behind the plunger may be generated for effecting the pumping action of the drug as will be described in more detail herein.

The drug container 6 may be of a conventional type of drug cartridge comprising a container having a barrel portion 6a, a neck portion 6b having an open end closed by a septum 6c, and a plunger 12 closing an open end of the barrel portion 6a, the liquid drug 78 to be administered to the patient being contained hermetically within the barrel portion 6a between the plunger and the septum. Such drug containers 6 are well-known in the pharmaceutical industry and may be used for containing in a sterile manner many different types of liquid drugs. Such drugs may also be provided in different sizes (volumes), it being understood that a drug delivery device according to embodiments of the present invention may be adjusted in dimensions to cater for different types of drug containers depending on the medical application. Embodiments of the invention may also be used with non-standard drug containers.

According to an aspect of the invention, in particular for single use and single injection of the contents of the drug container, for instance over time spanning a few minutes to 60 minutes, the drug delivery device incorporates a pneumatic pumping system in the drive unit 4 that pumps air to the drug container and applies pressure on the plunger 12 so as to advance the plunger and cause liquid 78 in the container to be pumped to the injection needle (not shown) once the drug delivery device has been activated.

According to an advantageous embodiment, the pneumatic pumping system may comprise a pump engine 28 having a design and configuration similar to the pump engine described in WO 2007074363 in which a rotor is mounted within a stator and is rotatably and axially movable within the stator in order to pump a fluid from a fluid inlet to a fluid outlet. As known from the above-mentioned publication, the rotor has a pump shaft with first and second diameters surrounded by seals that open and close a fluid channel between the inlet and outlet as the rotor rotates and axially displaces due to a cam mechanism between the stator and rotor, whereby during the opening and closing of the valves between the fluid inlet and pumping chamber, respectively between the pumping chamber and outlet, a pumping action is performed.

In summary, the pump engine 28 according to a preferred embodiment includes:

- a stator 28a,
- a rotor 28b slidably and rotatably mounted at least partially in the stator, the rotor comprising a first axial extension having a first diameter and a second axial extension having a second diameter greater than the first diameter,
- a first valve formed by a first valve seal mounted on the stator around the first axial extension, in conjunction with a first channel in the rotor that is configured to allow fluid communication across the first valve seal when the first valve is in an open position,
- a second valve formed by a second valve seal mounted on the stator around the second axial extension, in conjunction with a second channel in the rotor that is configured to allow fluid communication across the second valve seal when the second valve is an open position.

Although the pump engine design in general and the pumping action principle may be understood by referring to the above-mentioned publication, in embodiments of the present invention, the pumping system is not fluidically connected to the liquid to be administered. Rather, in the present invention the pump engine is used to pump air that creates a pressure on the container plunger 12 to displace the plunger and press liquid out of the container 6 via a septum needle 18 piercing through the septum 6c of the container 6.

An important advantage of using such a pump engine in embodiments of the present drug delivery device is that there is no direct connection of fluid between the inlet and outlet at any position of the rotor and no actuation of any valves are required, such that a particularly reliable pumping of gas without leakage is ensured in an easy to operate arrangement. The pump engine 28 is very compact and can be driven directly by a rotary electrical motor in the drive unit 4 without gearing. In effect, because of the differential pumping volume displacement that is defined by the axial displacement of the rotor and the difference between the first and second diameters of the pump shaft, the pumping volume displacement rotation can be easily configured for optimal operation with an electrical motor of a given type rotating with a constant speed. Moreover, the pump engine parts can be made entirely of polymer materials and the rotor may be easily coupled to the pump drive ensuring a sterile barrier between the fluidic portion of the pump engine and the coupling interface.

The drive unit 4 is configured principally to drive the pumping system 8, but may also have additional functions such as processing sensing signals, and transmitting and receiving data from an external device via a wireless communication link, for instance using Bluetooth.

The drive unit 4 comprises an electronic control system 47 that may include at least one microprocessor and optionally a wireless connection module. The electronic control system further comprises a power source for instance in the form of a battery 50, and an electrical motor 52 to drive the pump engine 28 of the pumping system 8.

The drive unit may further comprise a plunger sensing system 80 for sensing the position of the container plunger. The plunger sensing system is for determining correct operation of the drug delivery device and identifying for instance occlusion in the liquid flow system, or an end of travel of the plunger when the drug container is empty at the end of the drug administration process. Embodiments of the plunger sensing system 80 will be described in more detail further on.

The delivery unit comprises a container casing 38 having therein a container receiving cavity 75 into which the drug container 6 is inserted prior to assembly of the disposable delivery unit 3 to the reusable drive unit 4.

According to an aspect of the invention, the sealing adaptor 9 is configured to enclose a back end of the drug container in a hermetic manner such that a gas pressure within the drug container portion behind the plunger may be generated for effecting the pumping action of the drug.

The sealing adaptor is pluggably received on an end of the drug container facing an outer back side 73 of the plunger, the sealing adaptor comprising a sealing plug 23 inserted inside an end of the barrel portion 6a.

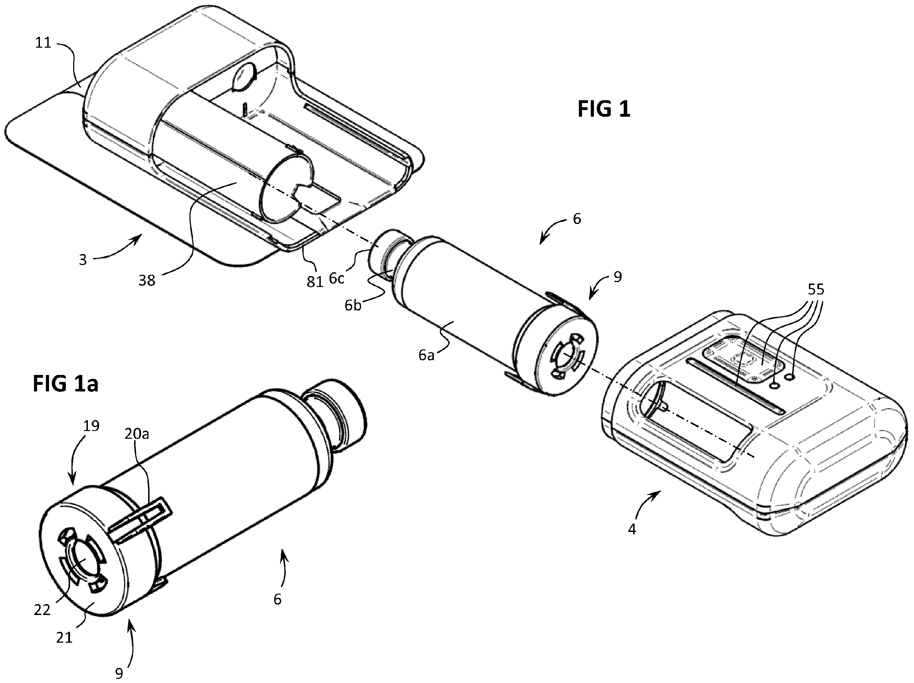

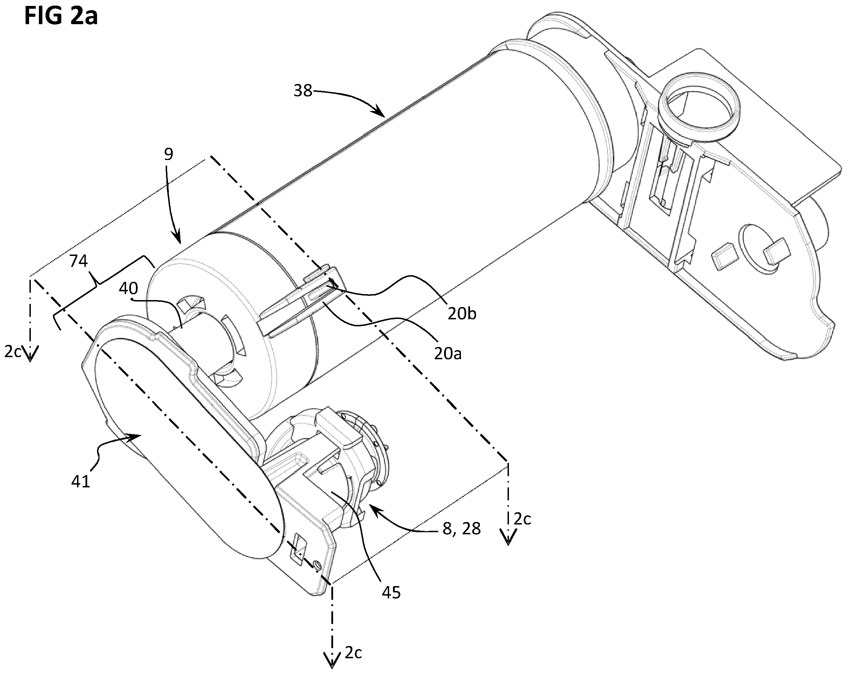

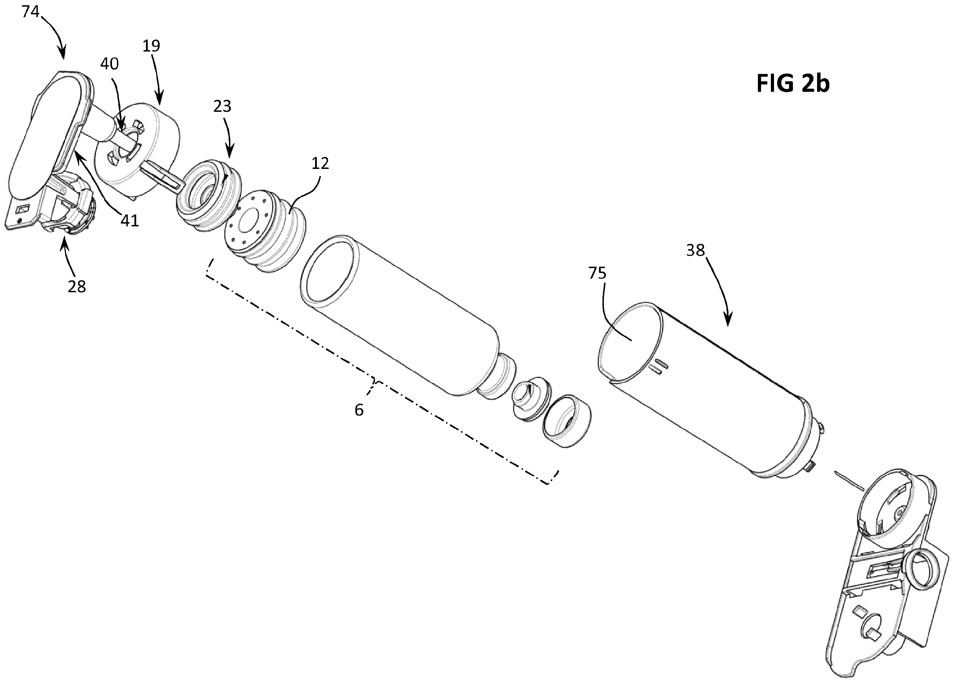

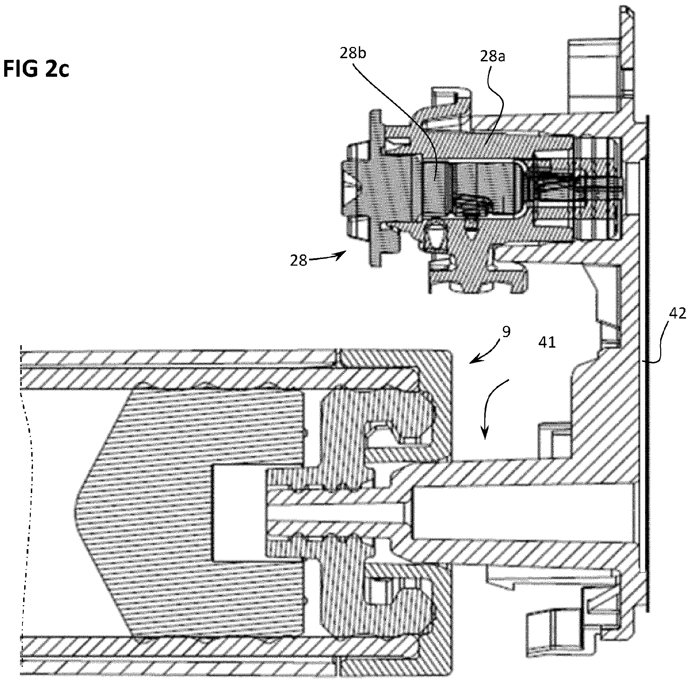

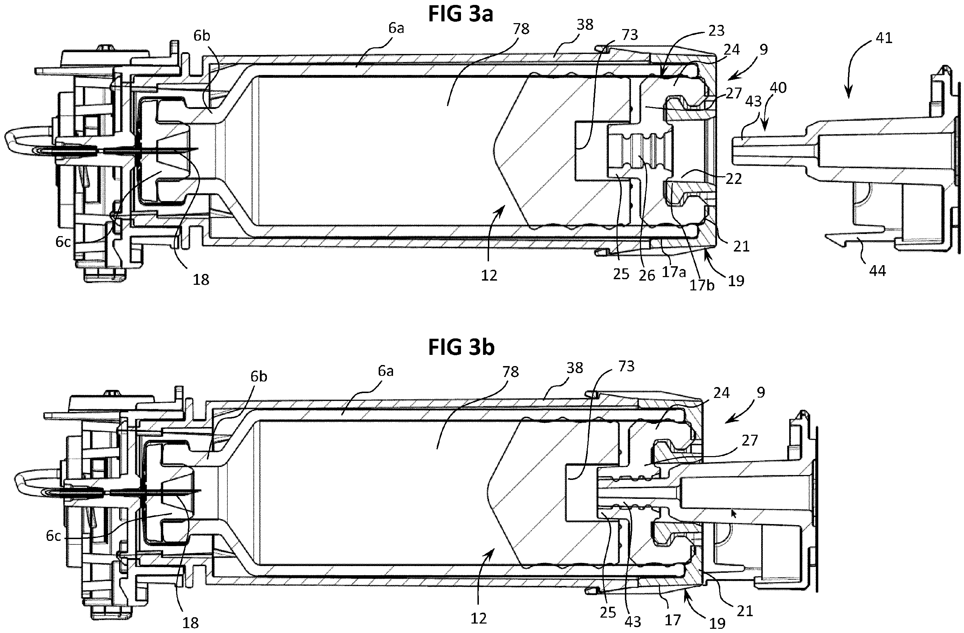

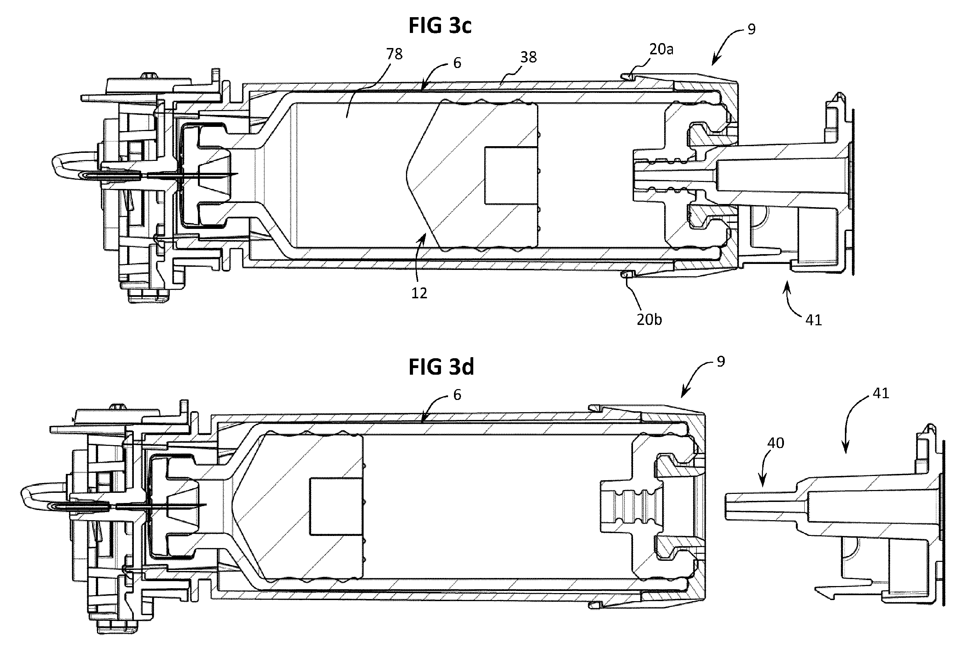

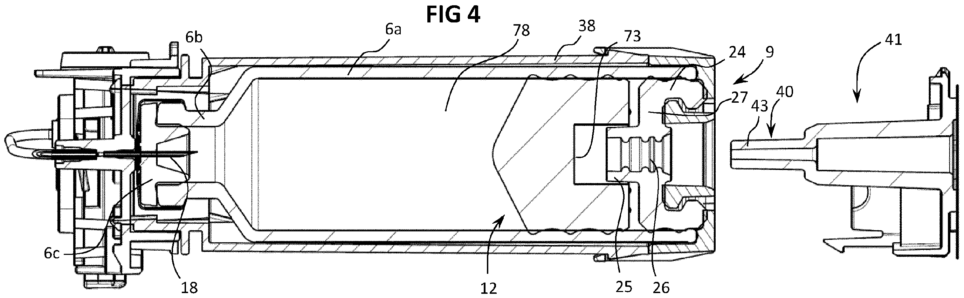

In an advantageous embodiment, as illustrated in figures 1-3c, the sealing adaptor may further comprise a cap 19 that mounts over an end of the barrel portion. That way, the force acting on the sealing plug 23 is retained by the container casing 38. In certain embodiments, it is however possible to not have a cap, as illustrated in figure 4. In this configuration, the force acting on the sealing plug 23 is transferred to the control / Drive Unit 4 and needs to be absorbed by the connection of the latter and the Delivery Unit 3.

The cap 19 includes an end wall 21 engaging a back end of the sealing plug, and one or more locking members 20a engaging the drug container or a housing component in which the drug container is received to securely hold the sealing plug in the container. In the illustrated embodiment, the locking member 20a engages a component of the delivery unit housing, in particular the container casing 38 which is provided with complementary locking members 20b. In a preferred embodiment, the locking member 20a comprises one or more elastically deformable latches that engage complementary locking shoulders 20b formed on the container casing outer surface. Various complementary locking elements with locking shoulders may however be provided.

The cap 19 of the sealing adaptor may comprise a circular channel between an outer ring wall 17a and an inner ring wall 17b that forms the central orifice 22, the outer ring wall 17a engaging over an outer end of the barrel portion of the drug container and the inner radial wall forming a space with the wall of the drug container within which an outer radial sealing ring 24 of the sealing plug is lodged.

In an embodiment, once the sealing adaptor 9 is assembled to the drug container and the drug container is assembled to the drug delivery device, the locking mechanism is preferably irreversible such the sealing adaptor may not be unplugged from the drug container.

The pneumatic flow system 74 fluidically interconnecting the pump engine 28 to the plunger, comprises a gas channel 42 formed in a channel housing component 41 mounted in the drive unit 4, the channel housing component 41 comprising a gas channel connector 40 configured for pluggable insertion in an orifice 26 extending through the sealing plug 23. In embodiments with a disposable delivery unit and reusable drive unit that may be coupled together for use and uncoupled after use, the plugging insertion of the gas channel connector in the sealing plug allows also unplugging after use.

The channel housing component 41 may be integrally molded or formed within a housing base or cover of the drive unit 4, or may be formed as a separate component that is assembled to the drive unit. In the latter case, the channel housing component 41 may comprise a first end 45 that engages sealingly over an outlet of the pump engine 28, and the other end branching across to the position of the drug container and presenting the gas connector 40 for insertion in the plugging orifice 26 of the sealing plug 23. The gas connector 40 comprises in a preferred embodiment a tube 43, preferably with a slightly tapered or conical outer surface, that is inserted into the sealing plug orifice 26, whereby compressible elastic sealing ribs provided around the plugging orifice 26 sealingly engaging the outer surface of the tube of the gas connector 40.

The sealing plug 23 of the sealing adaptor 9 may advantageously comprise an outer radial sealing ring 24 extending axially from the end wall 21 such that the outer radial sealing ring has more flexibility in the radial direction. The inner radial sealing ring 25 may also extend axially from the end wall 21 to provide more flexibility in the radial direction for the inner radial sealing ring. The sealing plug 23 may be made out of an elastomeric material similar to the material of the plunger 12, however it may also be made of a different compressible sealing material that that of the plunger since it does not require to be in contact with the liquid medicament and does not have a need to reduce a stick slip function within the drug container.

The plunger 12 may have a plunger back end 73 that forms a cavity surrounded by an outer plunger sealing ring wall 24 that also provides more flexibility for the sealing ring and allows insertion of the inner radial sealing ring 25 of the sealing plug 23 within the plunger back end cavity 73. This allows a more compact arrangement for a give container length allowing both the plunger and the sealing plug to be positioned at a rear end of the container barrel portion with a minimal reduction of the volume of medication 78 that can be filled in the drug container.

In order to have drug containers of a certain size, for instance a certain standard size, but to have a quantity of medication that may be less than the maximum volume for which the drug container is specified, the plunger 12 may be positioned along the barrel portion at the required distance from the septum 6c whereby the sealing plug 23 may have a length that is adjusted for the specified volume. This allows the volume behind the plunger into which the air under pressure is pumped to be initially small in order to reduce the volume of air that needs to be pumped by the pneumatic pumping system, considering that as the volume between the back end of the plunger and the sealing plug increases, a greater quantity of air needs to be pumped to maintain a given pressure.

The initial small volume of the space between the plunger and the sealing plug advantageously reduces the time required to build up the air pressure to the required operational pressure needed to move the plunger, which should overcome the stick-slip effect of the friction between the plunger and the drug container wall.

Advantageously, in the pneumatic drive configuration of embodiments of the invention, the pumping system is actuated to generate a gas pressure between the adaptor sealing plug 23 and plunger 12 to advance the plunger 12 during drug delivery. This configuration allows for a very compact delivery unit and therefore also of the drug delivery device 1, since very little space is required behind the plunger because there is no mechanical drive to directly push the plunger. Also, the use of a pump engine 28 as described above, *per se* known for pumping liquids, is particularly advantageous in the application of the pneumatic drive in view of the very compact size as well as the ability to pump gas without requiring additional valves. Moreover, the pump may be driven by an electrical motor without requiring gearing. Sterilization of the delivery unit 3 is also easy to perform as a substantially closed unit with gamma radiation, prior to assembly with the drug container 6.

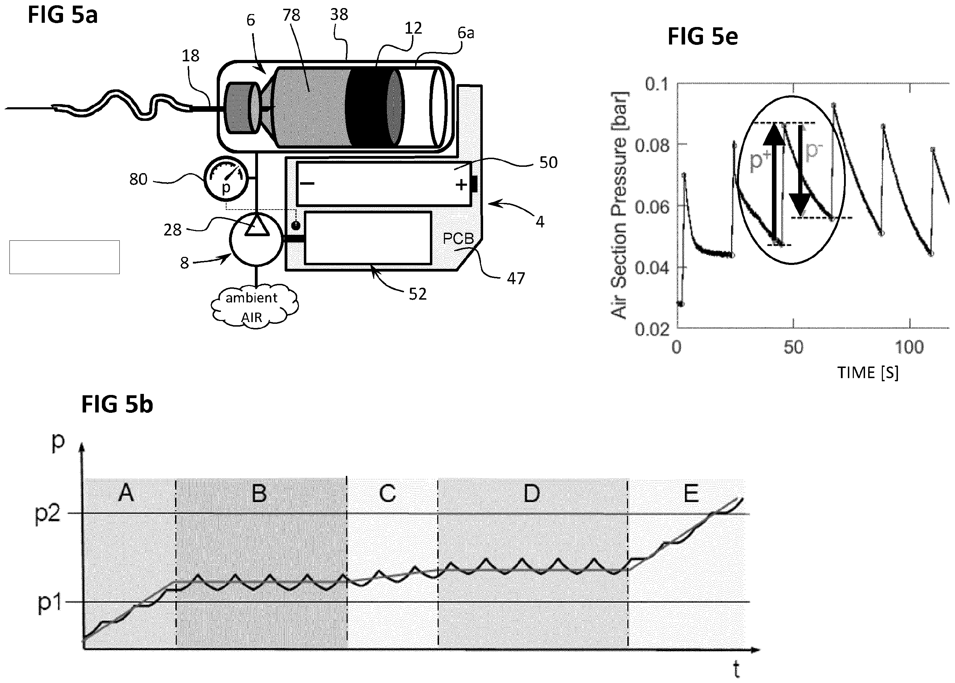

Referring to figures 5a to 5d, a drug delivery device with pneumatic flow system and pneumatic drive may comprise a pressure sensor 80 to measure the pressure in the pneumatic flow system 74. The pressure in the pneumatic flow system measured over time as illustrated in figure 5b is indicative of the displacement of the plunger 12. Blockage of the plunger due to occlusion in the liquid flow system may be detected by an augmentation in the rate of increase of the pressure over time. Also, the end of travel of the plunger, in other words the emptying of the drug container may also be easily detected for instance by a rise in the rate of increase in pressure identified in section *E* of figure 5b.

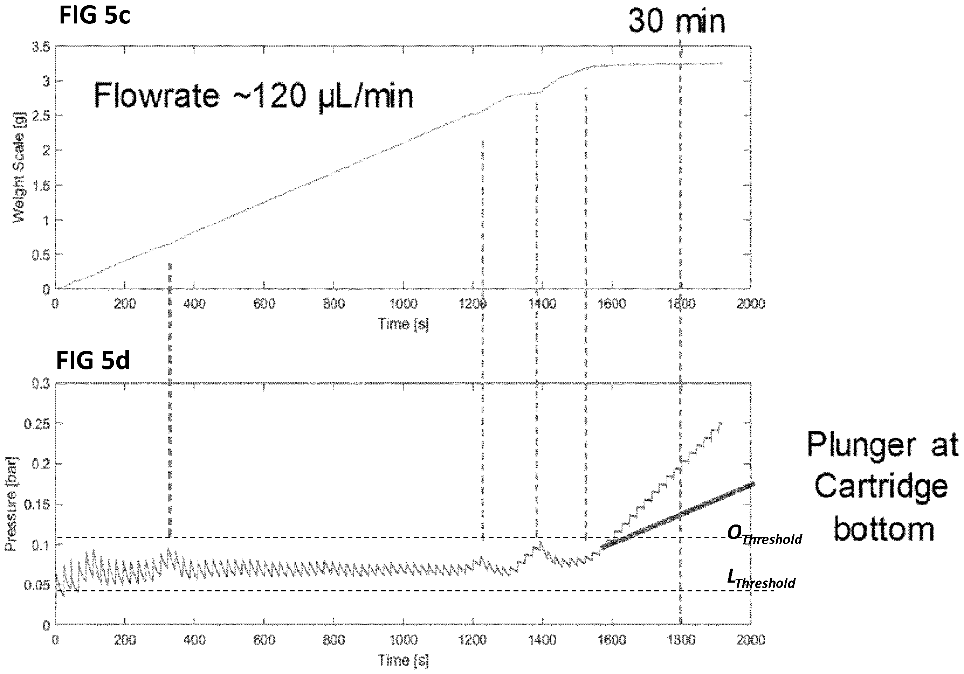

As illustrated in figure 5b, if a pneumatic pump system as described above is used, the air pumping action is preferably delivered in a pulsed manner, whereby there is a pumping phase followed by an inactive phase where the pump is stopped such that there is a variation in the air pressure that gives a saw tooth characteristic as illustrated in figures 5b and 5d. Each active pumping phase may be obtained by a single rotation cycle (360° rotation) of the pump engine rotor 32, or by a pre-defined plurality of rotation cycles. The inactive phase where the pump is stopped may be of a predefined duration, depending on the desired rate of drug delivery.

Evaluation of the pressure signal in the control unit 4 allows to obtain various information about the drug delivery device status, including:

> ➢ Current position of the plunger 12, thus current delivery volume → Algorithm 1
> ➢ Current speed of the plunger 12, thereby current delivery rate → Algorithm 2
> ➢ Detection of a delivery delay or a delivery standstill → Algorithm 3
> ➢ Detecting a leak in the air chamber → Algorithm 4

Examples of variants of the algorithms 1 to 4 are described below. The system information obtained from this can be processed by the control unit 4 of the pump system 8 alone or by correlating it with the feedback from other built-in sensors.

**Example of measurement method to determine current position of the plunger 12, thereby current dispensing volume (executed by an Algorithm 1 in the control unit)**

Referring to figures 5b, 5d and 5e, the amplitude $_p+,_p-$ of the pressure fluctuations decreases towards the end of delivery. As the plunger 12 progresses, the volume behind the plunger in the air section increases, whereby, the same volume of air is added per active pump phase. The ever-decreasing ratio of the added pump volume to the air section volume results, among other things, in a reduced increase in pressure during the active pumping phase $p+$. The position $x_{plunger}$ of the plunger 12, and thus also the volume of medication already dispensed $V_{drug}$, correlates reciprocally with this increase in pressure, according to Boyle-Mariotte's law:

$$x_{plunger} = 1/p+ * C0 = V_{drug} \qquad —$$

The constant $C_0$ depends on the air volume supplied per active pump phase and the initial volume (dead volume) of the air section $C_0$ can be easily determined experimentally.

**Example of measurement method to determine current speed of the plunger 12, hence current flow rate (executed by an Algorithm 2 in the control unit)**

Due to the pulsating operation of the pump, the pressure increase during the active pumping $p+$ phase can be compared with the pressure decrease during the inactive pumping phase $p-$ (i.e. the pause between active pump cycles $p+$). This enables a statement to be made about the speed of the plunger and the delivery rate.

$$p^-/p^+ * C1 = \dot{x}_{plunger} \quad \text{and} \quad p^-/p^+ * C2 = V_{drug}$$

If $p^-/p^+$ is within a cycle of pump operation and $p^-=1$, the volume of air delivered when the pressure increases corresponds to the volume of medication delivered when the pressure drops. In this state of equilibrium, the delivery rate of the drug corresponds to that of the compressed air from the pump. Deviations from the state of equilibrium are expressed in a deviation of the ratio $p^-/p^+$ from 1.

The constant $C_1$ represents the steady state plunger velocity and combines effects of friction, back pressure and pressure relative to the environment. It can be easily determined experimentally. The constant $C_2$ is calculated from the constant $C_1$ and the surface area of the plunger:

$$C_2 = C_1 * (D_{plunger})^2 * \pi / 4.$$

**Example of measurement method to determine detection of a plunger displacement delay or standstill (executed by an Algorithm 3 in the control unit)**

If the plunger 12 does not move or moves more slowly, the pressure in the air section behind the plunger increases. This behavior can be detected by a significant change in the ratio $p^-/p^+$ or the increase in the pressure level averaged over a longer period of time above an upper bound pressure threshold value $O_{Threshold}$

The stopping/slowing down of the plunger can have various causes:

➢ The delivery process is coming to an end, whereby the plunger 12 has reached the front end of the cartridge and the injection is finished;

➢ The delivery process is not completed, in which case the infusion needle is blocked (occlusion) or the plunger 12 is stuck, e.g. due to insufficient lubrication. In that case, issuing an alarm would be appropriate, for instance an acoustic, haptic or optic alarm;

**Example of measurement method to determine detection of a leak in the air section of the delivery unit (executed by an Algorithm 4 in the control unit)**

If the pump cannot build up any or only a low pressure in the air-filled section, this is most likely due to a leak. This can be detected by comparing the pressure level averaged over a certain period of time, for instance from 10 to 60 seconds, with a lower bound pressure threshold value $L_{Threshold}$.

A large amount of information can be obtained by using a single sensor which allows the system to be better monitored and made more secure. Excessive pressure, which could cause the cartridge to burst or be damaged, may be detected at an early stage.

Pressure sensors are very economical and easy to integrate. An advantage of this pneumatic plunger position sensing system is thus the very low cost and easy integration, and it is well-adapted in particular for a single injection cycle where control of the average flow rate, for instance as illustrated in figure 5c, is required and the end position of the plunger in the container should be determined. In such circumstances the accurate verification of the position of the plunger is not required. In such circumstances a very precise verification of the position of the plunger is not required, and for instance a positional measurement accuracy of plus or minus 10% to 20% is sufficient to detect end of delivery, occlusion, blockage, leakage, and the average rate of delivery required for the treatment. Although the accuracy of the pneumatic plunger position sensing system is lower than that of other sensing systems such as optical systems, certain treatment applications may advantageously benefit from the implementation of the pneumatic plunger position sensing system according to embodiments of this invention.

List of features

Drug delivery device 1

**Housing 2**

Skin contact wall 81
Needle orifice 10
Adhesive layer
Protective film 11

**Delivery unit 3**

**Drug 78**
**Drug container 6**

Barrel portion 6a
Neck portion 6b
Septum 6c
Plunger 12

Plunger back end 73
Plunger sealing ring wall 72

**Sealing adaptor 9**

Cap 19

Locking member 20a
e.g. latch arm
End wall 21
Orifice 22
Outer ring wall 17a
Inner ring wall 17b

Sealing plug 23

Outer radial sealing ring 24
Inner radial sealing ring 25
Plugging orifice 26
End wall 27

Container casing 38
Container receiving cavity 75
Locking member 20b
e.g. protrusion with shoulder
Septum needle 18
Pneumatic flow system 74

Connector 40

Tube 43
Locking element

Gas channel 42
Channel housing component 41

**Control unit or Drive unit 4**

**Pneumatic pumping system 8**

Pump engine 28

Stator 28a

Fluid inlet
Fluid outlet

Rotor 28b
Seals

Electronic control system 47

Circuit board
Microprocessor
Wireless connection module

Power source (battery) 50
Pump Motor 52
User interface 55
**Plunger sensing system 80**

**Claims**

1. A drug delivery device (1), comprising a delivery unit (3) including a drug container (6) comprising a barrel portion (6a) and a plunger (12) slidably mounted within the barrel portion and sealing the drug (78) within the container at one end of the barrel portion, and a drive unit (4) comprising a pneumatic flow system (74) and a pneumatic pumping system configured to pump air via the pneumatic flow system to a space behind the plunger to generate a gas pressure to advance the plunger in the container during drug delivery, **characterized in that** the drug delivery device comprises a sealing adaptor (9) mounted on an end of the drug container facing an outer side of the plunger, the sealing adaptor comprising a sealing plug (23) inserted inside an end of the barrel portion, the sealing plug comprising an orifice (26) configured for pluggable sealing connection to a gas channel connector (40) of the pneumatic flow system.

2. The drug delivery device according to the preceding claim wherein the gas channel connector (40) comprises a tubular end portion pluggably inserted in the orifice (26) of the sealing plug.

3. The drug delivery device according to any preceding claim wherein the sealing plug comprises an inner radial sealing ring (25) comprising a portion extending axially from an end wall (27) of the sealing plug, said orifice for sealing connection to the gas channel connector being formed within the inner radial sealing ring.

4. The drug delivery device according to the preceding claim wherein the plunger (12) comprises a back end (73) forming a cavity, said axial extension of the inner radial sealing ring being positioned at least partially in the cavity in an initial position prior to drug delivery.

5. The drug delivery device according to any preceding claim wherein the sealing plug comprises an outer radial sealing ring (24) comprising a portion extending axially from an end wall (27) of the sealing plug, the outer radial sealing ring sealingly engaging an inner surface of the barrel portion of the drug container.

6. The drug delivery device according to any preceding claim wherein the sealing adaptor comprises a cap (19) inserted over an end of the barrel portion, the cap comprising an end wall engaging a back end of the sealing plug and one or more locking members (20) engaging a housing component of the delivery unit in which the drug container is received.

7. The drug delivery device according to the preceding claim wherein the housing component of the delivery unit is a container casing (38) within which the drug container is inserted in the delivery unit (3), the locking member of the sealing adaptor cap engaging the complementary locking member on the container casing.

8. The drug delivery device according to the preceding claim wherein the one or more locking members on the cap comprises one or more elastic latches having a locking shoulder engaging a complementary locking shoulder on the outside of the container casing.

9. The drug delivery device according to any of the three directly preceding claims wherein the cap of the sealing adaptor comprises a circular channel between an outer ring wall (17a) and an inner ring wall (17b), the outer ring wall engaging over an outer end of the barrel portion of the drug container and the inner radial wall forming a space with the wall of the drug container within which an outer radial sealing ring (24) of the sealing plug is lodged.

10. The drug delivery device according to any preceding claim wherein the pneumatic flow system (74) comprises a channel housing component (41) within which a gas channel (42) is formed extending between the gas channel connector (40) and the outlet of the pump engine (28).

11. The drug delivery device according to the preceding claim wherein the gas channel housing component (41) is formed as a separated part assembled in the drive unit (4) to the pump engine.

12. The drug delivery device according to any preceding claim, wherein the pneumatic pumping system comprises a pump engine (28) including

    - a stator (28a),
    - a rotor (28b) rotatably and axially slidably mounted at least partially in the stator, the rotor comprising a first axial extension having a first diameter and the second axial extension having a second diameter greater than the first diameter,

- a first valve formed by a first valve seal mounted on the stator around the first axial extension, in conjunction with a first channel in the rotor that is configured to allow fluidic communication across the first valve seal when the first valve is in an open position, and

- a second valve formed by a second valve seal mounted on the stator around the second axial extension, in conjunction with a second channel in a rotor that is configured to allow fluidic communication across the second valve seal when the second valve is an open position.

**13.** The drug delivery device according to any preceding claim, wherein the drug container is a drug cartridge and comprises a septum (6c) at one end.

**14.** The drug delivery device according to any preceding claim, wherein the drive unit comprises an electronic control system and a pressure sensor (80) fluidically coupled to pneumatic flow system for measuring a gas pressure behind the plunger, the pressure sensor connected to the electronic control system (47) of the drive unit configured to measure a pressure detected by the pressure sensor over time and to determine from the pressure measurement over time a position of the plunger over time, including a stop in movement of the plunger either due to occlusion in the drug delivery flow system or an end of travel of the plunger within the container corresponding to a container empty position.

FIG 1

FIG 1a

# FIG 2a

EP 4 338 770 A1

FIG 2b

FIG 2c

**FIG 3a**

**FIG 3b**

EP 4 338 770 A1

**FIG 3c**

**FIG 3d**

**FIG 4**

**FIG 5a**

**FIG 5e**

**FIG 5b**

FIG 5c

30 min

Flowrate ~120 µL/min

FIG 5d

Plunger at Cartridge bottom

$O_{Threshold}$

$L_{Threshold}$

EP 4 338 770 A1

Europäisches
Patentamt

European
Patent Office

Office européen
des brevets

# EUROPEAN SEARCH REPORT

**Application Number**

EP 22 19 5657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/249778 A1 (MOJARRAD MEHRAN [US] ET AL) 11 August 2022 (2022-08-11) * paragraphs [0037], [0043] * * figures 7A,B * | 1-14 | INV. A61M5/142 A61M5/145 ADD. A61M5/168 |
| X | US 2020/164155 A1 (MOJARRAD MEHRAN [US] ET AL) 28 May 2020 (2020-05-28) * paragraphs [0053] – [0059] * * figures 1, 5A,B * | 1-14 | |
| X | US 2015/335817 A1 (LAMBERT PAUL [US]) 26 November 2015 (2015-11-26) * figures 1,2 * | 1-14 | |
| A | US 2009/123309 A1 (HILBER JOSEF [CH] ET AL) 14 May 2009 (2009-05-14) * paragraphs [0001], [0009] * * figure 2 * | 1-14 | |
| A | US 5 433 704 A (ROSS STEPHEN O [US] ET AL) 18 July 1995 (1995-07-18) * column 11, line 67 – column 12, line 1 * | 1-14 | TECHNICAL FIELDS SEARCHED (IPC) A61M |

The present search report has been drawn up for all claims

| Place of search | Date of completion of the search | Examiner |
|---|---|---|
| Munich | 7 February 2023 | Walther, Manuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 5657

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2023

| Patent document cited in search report | | Publication date | Patent family member(s) | | Publication date |
|---|---|---|---|---|---|
| US 2022249778 | A1 | 11-08-2022 | US 2022249778 | A1 | 11-08-2022 |
| | | | WO 2021011717 | A1 | 21-01-2021 |
| US 2020164155 | A1 | 28-05-2020 | EP 3664863 | A2 | 17-06-2020 |
| | | | MA 49838 | A | 17-06-2020 |
| | | | US 2020164155 | A1 | 28-05-2020 |
| | | | WO 2019032482 | A2 | 14-02-2019 |
| US 2015335817 | A1 | 26-11-2015 | NONE | | |
| US 2009123309 | A1 | 14-05-2009 | AT 409280 | T | 15-10-2008 |
| | | | AT 447106 | T | 15-11-2009 |
| | | | AU 2006329605 | A1 | 05-07-2007 |
| | | | BR PI0620741 | A2 | 18-09-2012 |
| | | | CA 2669637 | A1 | 05-07-2007 |
| | | | CA 2844349 | A1 | 05-07-2007 |
| | | | CN 101351642 | A | 21-01-2009 |
| | | | CN 102691637 | A | 26-09-2012 |
| | | | DK 1803934 | T3 | 02-02-2009 |
| | | | DK 1966490 | T3 | 18-01-2010 |
| | | | DK 2143947 | T3 | 11-02-2013 |
| | | | EP 1803934 | A1 | 04-07-2007 |
| | | | EP 1966490 | A2 | 10-09-2008 |
| | | | EP 2143947 | A1 | 13-01-2010 |
| | | | ES 2313266 | T3 | 01-03-2009 |
| | | | ES 2334459 | T3 | 10-03-2010 |
| | | | ES 2397806 | T3 | 11-03-2013 |
| | | | HK 1125686 | A1 | 14-08-2009 |
| | | | IL 192186 | A | 31-01-2012 |
| | | | IL 214359 | A | 31-12-2012 |
| | | | JP 5115742 | B2 | 09-01-2013 |
| | | | JP 2009521969 | A | 11-06-2009 |
| | | | KR 20080078861 | A | 28-08-2008 |
| | | | KR 20130099259 | A | 05-09-2013 |
| | | | PL 1803934 | T3 | 31-03-2009 |
| | | | PL 1966490 | T3 | 31-03-2010 |
| | | | PL 2143947 | T3 | 29-03-2013 |
| | | | RU 2011122665 | A | 10-12-2012 |
| | | | US 2009123309 | A1 | 14-05-2009 |
| | | | WO 2007074363 | A2 | 05-07-2007 |
| | | | ZA 200805113 | B | 27-05-2009 |
| US 5433704 | A | 18-07-1995 | US 5207645 | A | 04-05-1993 |
| | | | US 5308335 | A | 03-05-1994 |
| | | | US 5433704 | A | 18-07-1995 |
| | | | US 5584811 | A | 17-12-1996 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 19 5657

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2023

| Patent document cited in search report | Publication date | Patent family member(s) | Publication date |
|---|---|---|---|
| | | US 5743878 A | 28-04-1998 |
| | | WO 9409847 A1 | 11-05-1994 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007074363 A